Europäisches Patentamt

European Patent Office

Office européen des brevets

(11) Veröffentlichungsnummer: **0 345 588**
**A1**

## (12) EUROPÄISCHE PATENTANMELDUNG

(21) Anmeldenummer: **89109652.1**

(22) Anmeldetag: **29.05.89**

(51) Int. Cl.4: **C12M 1/00 , C12P 1/00 , C12M 1/36**

(30) Priorität: **03.06.88 DE 3818988**

(43) Veröffentlichungstag der Anmeldung:
**13.12.89 Patentblatt 89/50**

(84) Benannte Vertragsstaaten:
**CH DE FR GB IT LI NL SE**

(71) Anmelder: **Gesellschaft für
Biotechnologische Forschung mbH (GBF)
Mascheroder Weg 1
D-3300 Braunschweig-Stöckheim(DE)**

(72) Erfinder: **Bellgardt, K.-H., Dr. Dipl.-Biol.
Mascheroder Weg 1
D-3300 Braunschweig(DE)**
Erfinder: **Afschar, A.S., Dr. Dipl.-Biol.
Mascheroder Weg 1
D-3300 Braunschweig(DE)**

(74) Vertreter: **Boeters, Hans Dietrich, Dr. et al
Thomas-Wimmer-Ring 14
D-8000 München 22(DE)**

(54) **Verfahren und Anordnung zur Regelung eines biologischen Wachstumsprozesses.**

(57) Bei einem biologischen Wachstumsprozeß wird die Substratzufuhr dadurch geregelt, daß in einer Folge von Zyklen (Z) wiederholt pulsförmig Substrat in einen Bioreaktor (BR) gegeben wird. Ein Zyklus (Z) besteht aus einem Zeitabschnitt (T1), in dem der Substratfluß vergleichsweise hohe Werte besetzt und einem folgenden Zeitabschnitt (T2), in dem der Substratfluß vergleichsweise geringe Werte besitzt. Zur Festlegung des Substratflusses werden die in einem Zyklus auftretenden Wachstumsphasen über ein logisches Modell ermittelt.

FIG.1

EP 0 345 588 A1

## Verfahren und Anordnung zur Regelung eines biologischen Wachstumsprozesses.

Die Erfindung betrifft ein Verfahren und eine Anordnung zur Regelung eines biologischen Wachstumsprozesses gemäß den Oberbegriffen der Patentansprüche 1 und 5.

Bei vielen biotechnologischen Prozessen ist es notwendig, kontinuierlich oder wiederholt in bestimmten Zeitabschnitten Substrat (Kohlenstoffquelle) oder andere Substanzen, die von den Mikroorganismen aufgenommen werden und die im folgenden vereinfacht als Substrate bezeichnet werden, in den Reaktor zu geben. Um hohe Ausbeuten oder Produktivität zu erreichen, muß die Substratgabe in ihrem zeitlichen Verlauf an variable Eigenschaften der Mikroorganismen, Schwankungen der Substratqualität, sich verändernden Konzentrationen und Betriebsparametern im Reaktor angepaßt werden.

Bei biologischen Wachstumsprozessen ist es bisher üblich, bei der Zuführung von Substrat zum Reaktor kontinuierliche Regelstrategien zu verwenden, d.h. Strategien, bei denen das Substrat abhängig vom Verlauf des Wachstumsprozesses dem Reaktor kontinuierlich zugeführt wird.

Aufgabe der Erfindung ist es, ein Verfahren und eine Anordnung zur Regelung eines biologischen Wachstumsprozesses bereit zu stellen, das in flexibler Weise bei hoher Betriebssicherheit eine Regelung des biologischen Wachstumsprozesses ermöglicht.

Insbesondere soll das Verfahren und Anordnung so ausgestaltet sein, daß eine Automatisierung der Substratzugabe in den Reaktor möglich ist.

Diese Aufgabe wird bei einem Verfahren und einer Anordnung der eingangs genannten Art dadurch gelöst, daß die Zuführung des Substrates zum Reaktor impulsweise geregelt in einer Folge von Zyklen erfolgt, die aus Zeitabschnitten mit relativ hohem Substratfluß und Zeitabschnitten mit relativ niederen Substratfluß bestehen. Mittels eines logischen Modelles des Wachstumsprozesses werden dabei automatisch während eines Zyklus die Wachstumsphasen des Mikroorganismus erkannt und in Abhängigkeit davon wird der Wachstumsprozeß geregelt.

Bei einer vorteilhaften Ausführungsform der Erfindung werden die Wachstumsphasen über die Übergänge von einer Wachstumsphase zur anderen erkannt.

Der Pulsverlauf der Substratzufuhr wird dabei in Abhängigkeit von den charakteristischen Größen des Wachstumsprozesses und/oder der Art des Mikroorganismus festgelegt.

Die erfindungsgemäße Anordnung weist einen Regelkreis auf, der mittels logischer Strukturen durch Vergleich eines Wachstumsprozeßmodelles mit dem tatsächlichen Verlauf des Wachstumsprozesses den Wachstumsprozeß regelt.

Der Regelkreis enthält dabei eine Fühleinrichtung zum Erfassen von prozeßrelevanten Meßgrößen, einen Analysen- und Entscheidungsblock zum Erfassen von charakteristischen Wachstumsphasen des Mikroorganismus über eine logische Modellstruktur während eines Zyklus und einen Regelungsblock, der in Abhängigkeit von dem Verlauf der Wachstumsphasen über die Fördereinrichtung die Substratzufuhr regelt.

Weitere vorteilhafte Ausführungsformen der Erfindung sind in den Unteransprüchen gekennzeichnet.

Ausgehend von dem Pulszufütterungsprinzip ergeben sich neue Möglichkeiten und vorteilhafte Eigenschaften, die mit üblichen, kontinuierlichen Regelstrategien nicht oder nur schwer erzielbar sind. Diese sind:

Ausschließliche Verwendung von einfach zugänglichen Meßgrössen, wie Abgaskonzentrationen von $O_2$ und $CO_2$, pH, $PO_2$, Fluoreszenz, usw.

Unkritische Anforderungen an die Meßgenauigkeit, da die Regelung während eines Zyklus hauptsächlich die Tendenz und das prinzipielle zeitliche Muster der Meßgrößen auswertet.

Flexible Wahl der Meßgrößen bei ähnlichem zeitlichen Muster. Dies erhöt erheblich die Betriebssicherheit, da auch während des laufenden Prozesses auf eine andere Meßgröße umgeschaltet werden kann, z.B. vom pH auf $PO_2$.

Die Anzahl der tatsächlich gleichzeitig für die Regelung benutzten Meßgrößen ist vom jeweiligen Prozeß abhängig. Es ist eine besondere vorteilhafte Eigenschaft des erfindungsgemäßen Verfahrens, daß auch bei sehr komplexen Wachstumsprozessen normalerweise eine einzige, geeignet gewählte Meßgröße zur sicheren Regelung ausreicht. Zur Erhöhung der Betriebssicherheit oder der Regelgeschwindigkeit kann jedoch die Heranziehung weiterer Meßgrößen und deren gleichzeitige Auswertung in den logischen Modellstrukturen vorteilhaft sein.

Bei den angegebenen Beispielen wurden maximal zwei Meßgrößen benutzt. Diese Zahl ist jedoch kein fester Parameter des Verfahrens.

Adaption an variable biologische Eigenschaften, wie Atmungsaktivität oder maximale Wachstumsrate.

Betrieb in labilen biologischen Zuständen.

Vereinfachte Schätzung biologischer Parameter, wie Ausbeute, spezifische Wachstumsrate oder Zellkonzentration.

Die verwendete Regelungsstrategie läßt sich in vorteilhafter Weise auch bei großbiotechnologischen Prozessen verwenden.

Das erfindungsgemäße Verfahren ermöglicht eine hohe Produktivität und Ausbeute.

Ausführungsformen der Erfindung sind in den Zeichnungen dargestellt und werden im folgenden beispielsweise näher beschrieben. Es zeigen:

Fig. 1 eine schematische Darstellung eines gemäß der Erfindung geregelten Wachstumsprozesses.

Fig. 2 bis 4 verschiedene Betriebsarten des geregelten Wachstumsprozesses.

Fig. 5 ein Blockdiagramm des Gesamtsystems.

Fig. 6 eine schematische Darstellung des dem Analysen- und Entscheidungsblock zugrunde liegenden logischen Modells, mit der dazugehörigen Ablauffolge der Regelungsphasen.

Fig. 7 ein schematisches Blockdiagramm des Analysen- und Entscheidungsblockes.

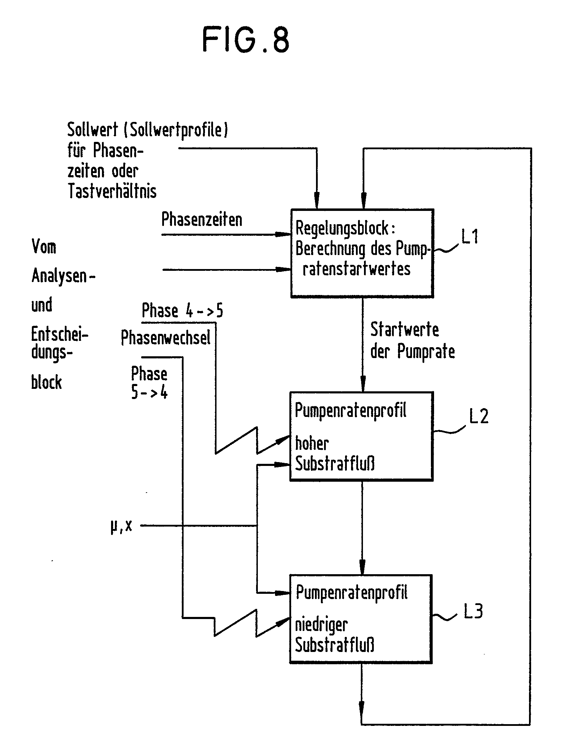

Fig. 8 ein schematisches Blockdiagramm des Regelungsblockes.

Fig. 9 ein Anwendungsbeispiel des erfindungsgemäßen Systems bei der Hefekultivierung im Chemostaten.

Fig. 10 und 11 ein Anwendungsbeispiel des erfindungsgemäßen Systems bei der Backhefekultivierung im Fed-Batch und

Fig. 12 und 13 ein Anwendungsbeispiel des erfindungsgemäßen Systems in der Fed-Batch Produktion von 2,3-Butandiol.

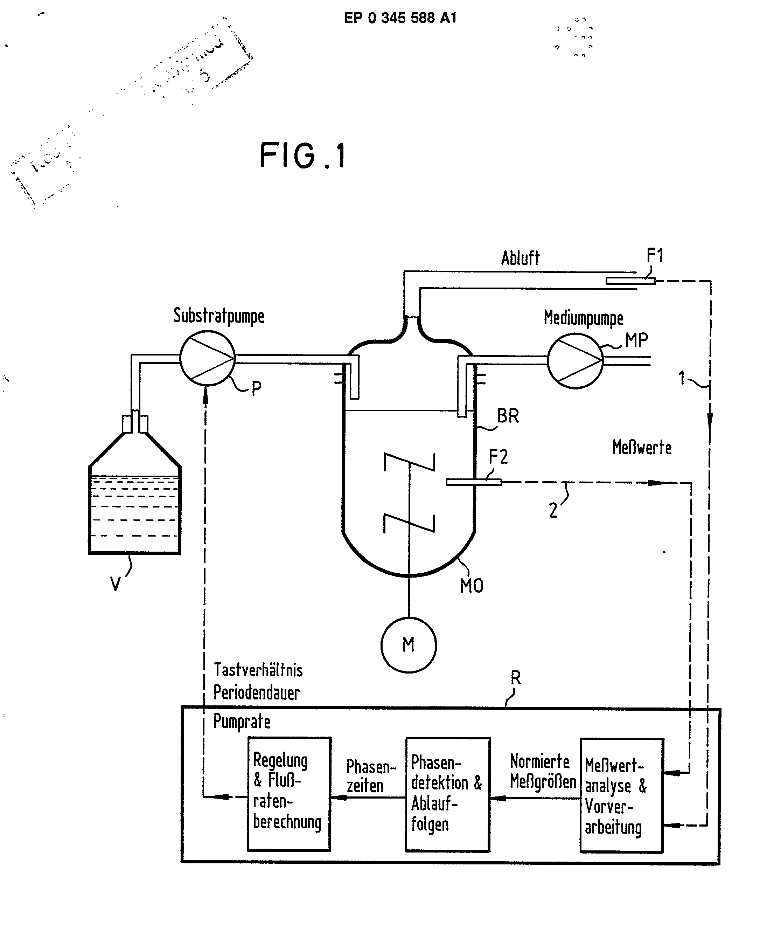

Bei einem in der Fig. 1 dargestellten biotechnologischen Prozeß wird in einem Bioreaktor BR ein Mikroorganismus MO gezüchtet. Der Reaktor kann in bekannter Weise ein motorisch angetriebenes Rührwerk M aufweisen. Über eine Substratpumpe P wird dem Reaktor BR aus einem Vorratsbehälter V Substrat, z.B. Zuckermelasse zugeführt, das von den Mikroorganismen aufgenommen wird. Eine Mediumpumpe MP ermöglicht es, die Zellmasse im Reaktor BR abzusaugen. Zur Verfolgung von Prozeßrelevanten Größen sind Fühlelemente (Meßwertgeber) F1 und F2 angeordnet, wobei der Meßwertgeber F1 eine Meßgröße 1 erfaßt, die z.B. die Kohlendioxydkonzentration im Abgas sein kann. Der Meßwertgeber F2 erfaßt die Meßgröße 2, die der pH-Wert im Medium sein kann. Diese Meßwerte werden einer Regelanordnung R zugeführt. Die Regelanordnung R regelt die Substratzufuhr über die Substratpumpe P.

Für biologische Wachstumsprozesse gibt es bekanntlich verschiedene Prozeßführungsarten. Diese lassen sich im wesentlichen wie folgt charakterisieren: im Batch-Betrieb, Fed-Batch-Betrieb und kontinuierlicher oder Chemostat-Betrieb.

Der Batch-Betrieb ist eine Prozeßführung, bei der nur einmalig zu Prozeßbeginn Substrat mit einer relativ hohen Konzentration im Fermenter vorgelegt wird. Der Batch-Prozeß ist im Prinzip beendet, wenn die Mikroorganismen das Substrat vollständig oder soweit wie ihnen möglich ist, verbraucht haben.

Fed-Batch-Betrieb ist eine Prozeßführung, bei der kontinuierlich Substrat in der Fermenter gegeben wird, ohne jedoch entsprechende Mengen an Medium abzuziehen, so daß sich das Volumen laufend erhöht. Beim Fed-Batch-Betrieb ist die Substratkonzentration im Reaktor üblicherweise sehr gering.

Kontinuierlicher oder Chemostatbetrieb ist eine Prozeßführung, bei der kontinuierlich Substrat in den Fermenter gegeben und gleichzeitig eine entsprechende Menge an Medium abgezogen wird, so daß das Volumen konstant bleibt. Beim kontinuierlichen Betrieb ist die Substratkonzentration im Reaktor üblicherweise gering.

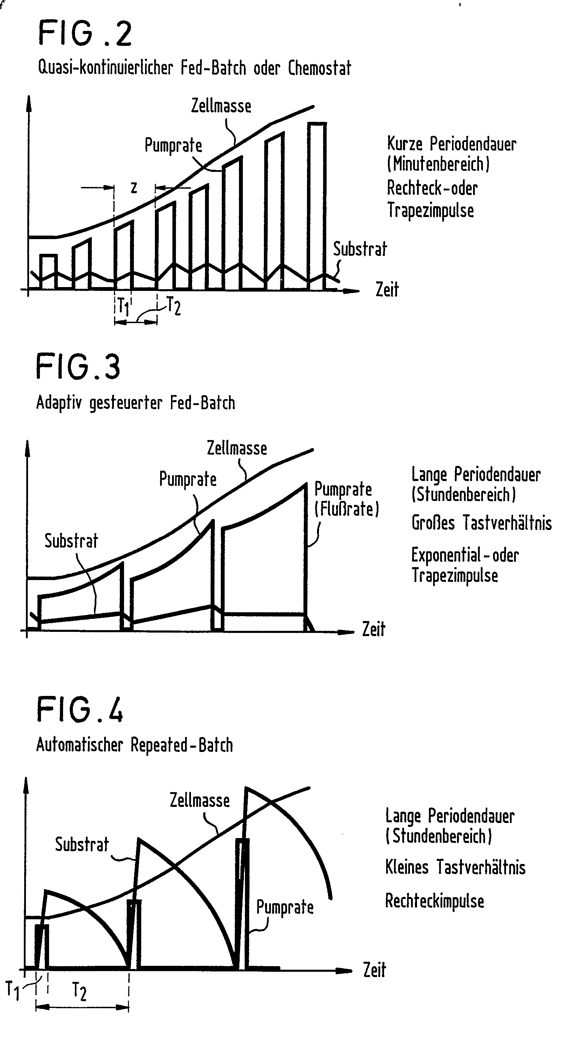

Entsprechend den Darstellungen der Fig. 2 bis 4 können bei dem erfindungsgemäßen Prozeß in der Betriebsphase u.a. die dargestellten Prozeßführungsarten verwendet werden. Dabei bezeichnet gemäß Fig. 2 der quasi-kontinuierlich geregelte Fed-Batch oder Chemostatprozeß (vereinfachend als quasi-kontinuierlicher Fed-Batch oder Chemostat bezeichnet) eine Betriebsart, bei der das Substrat nicht kontinuierlich, sondern pulsweise zugegeben wird. Die Pulsperiode ist jedoch dabei so klein, daß das Verhalten des Prozesses und die der Regelung praktisch als kontinuierlich angesehen werden können. Beim Chemostat wird zur Unterscheidung vom normalen kontinuierlichen Betrieb die Bezeichnung Pulsostat verwendet.

Adaptiv gesteuerter Fed-Batch- oder Chemostatprozeß entsprechend der Fig. 3 ist eine Betriebsart, bei der das Substrat in längeren Zeitabschnitten kontinuierlich entsprechend der gewählten Steuerfunktion zugegeben wird (Open-Loop-Phase), ohne daß ein Eingriff durch die Regelung stattfindet. Danach wird der Substratfluß kurzzeitig unterbrochen. Beide Phasen bilden zusammen die Pulsperiode, im folgenden als Zyklus bezeichnet. Beide Phasen werden, wie später beschrieben, in der Regelungsanordnung mittels eines logischen Modelles verfolgt. Die Regelung erkennt daher den Limitierungsgrad der Kultur und kann eine Neuberechnung der Flußrate für den nächsten Zyklus vornehmen. Die Open-Loop-Steuerfunktion wird also periodisch an den tatsächlichen Prozeßverlauf adaptiert. Beim Chemostat wird zur Unterscheidung vom normalen kontinuierlichen Betrieb die Bezeichnung semikontinuierlicher Chemostat verwendet.

Der automatische Repeated-Batch entsprechend der Fig. 4 ist eine Betriebsart, bei der mehrere Batch-Zyklen zeitlich nacheinander gefahren werden und zwar dadurch, daß man am Ende eines Batch-Zyklus durch Zugabe von Substrat in einem kurzen Zeitraum die Substratkonzentration wieder auf hohe Werte einstellt. Danach wächst die Kultur über einen längeren Zeitraum, bis sie wieder in die Substratlimitierung übergeht. Beide Phasen bilden zusammen die Pulsperiode. Sie werden wie ebenfalls später beschrieben, durch die Regelungsanordnung bestimmt. Die Regelung erkennt daher den Limitierungszeitpunkt der Kultur und kann die Neuzugabe des Substrates für den nächsten Zyklus initiieren. Die zugegebene Menge von Substrat kann dabei jeweils konstant bleiben oder mittels der Regelungseinrichtung an den Prozeßverlauf angepaßt werden.

Bei Repeated-Batch wird also durch die Regelung zyklisch Substrat in den Reaktor gegeben. Zusätzlich kann jedoch vor der erneuten Zugabe des Substrates eine bestimmte Menge des Mediums aus dem Reaktor entnommen werden, entweder durch die Medienpumpe, durch einfaches Ablassen, oder durch eine andere geeignete Vorrichtung. Dieser Vorgang wird ebenfalls vom Regelungsblock kontrolliert, derart daß das Medienvolumen im Reaktor in jedem Zyklus gleich bleibt oder einem vorgegebenen Verlauf folgt.

Gemeinsam jedoch für die dargestellten Prozeßführungsarten ist die erfindungsgemäße Regelungsstrategie, bei der in einer Folge von Zyklen (Z) wiederholt pulsförmig Substrat in den Bioreaktor BR gegeben wird. Ein Zyklus bzw. Pulszyklus (Fig. 2 bis 4) besteht aus einem Zeitabschnitt T1, in dem der Substratfluß vergleichweise hohe Werte besitzt, und einem folgenden Zeitabschnitt T2, in dem der Substratfluß vergleichsweise geringe Werte besitzt. Als spezielle Realisierung kann der Substratfluß in diesem Zeitabschnitt auch ganz ausgeschaltet sein. Der Zeitliche Verlauf der Flußrate in den beiden Zeitabschnitten wird wie später erläutert, über die Regelung bestimmt. Das Verhältnis von T1 zu T1 und T2 wird als Tastverhältnis bezeichnet, die Summe von T1 plus T2 als Periodendauer der Pulszyklen. Die Größenordnung der Zeiten T1 und T2 wird nach den Erfordernissen des biologischen Systems, der gewünschten Art der Prozeßführung und den gewünschten Regeleigenschaften festgelegt. Diese Größenordnungen sind daher Vorgabeparameter der Regelung. Anhaltswerte für die verschiedenen Prozeßführungsarten gemäß den Fig. 2 bis 4 sind die folgenden:

Quasi-kontinuierlich geregelter Fed-Batch: T1, T2 Minutenbereich bis 10-Minutenbereich.

Quasi-kontinuierlich geregelter Chemostat (Pulsostat): T1, T2 Minutenbereich bis 10-Minutenbereich.

Adaptiv gesteuerter Fed-Batch: T1 = Stundenbereich, T2 = 10-Minutenbereich.

Adaptiv gesteuerter (semikontinuierlicher) Chemostat: T1 = Stundenbereich, T2 = 10-Minutenbereich.

Repeated-Batch: T1 = Sekunden- bis Minutenbereich, T2 = Stundenbereich.

Die konkreten Zeiten werden von der Regelung anhand des momentanen dynamischen Verhaltens der Regelstrecke (Bioreaktor und Kultur) bestimmt und können nach unten oder oben abweichen.

Während eines Zyklus Z durchläuft die Kultur verschiedene Wachstumsphasen. Eine Wachstumsphase ist dabei eine Zeitabschnitt mit einer bestimmten Stoffwechselaktivität der Kultur. Die verschiedenen Wachstumsphasen werden nach den jeweils überwiegenden Stoffwechselreaktionen unterschieden, z.B. Substratverbrauch zur Zellmassenproduktion oder zur Synthese eines Produktes, oxidativer Stoffwechsel mit hoher Atmungsaktivität, fermentativer Stoffwechsel mit niedriger Atmungsaktivität, Stoffwechsel zur Erhaltung der Zellstruktur, Anpassung des Stoffwechsels an veränderte Fermentationsbedingungen usw. Welche Wachstumsphasen bei einem konkreten Prozeß vorhanden sind, richtet sich nach den eingesetzten Mikroorganismen und der jeweiligen Prozeßführung. Bei den hier vorgeschlagenen Regelungskonzept sind mindestens zwei Wachstumsphasen vorhanden, nämlich Stoffwechsel bei hoher Substratversorgung und Stoffwechsel bei niedriger Substratversorgung oder Substratunterversorgung.

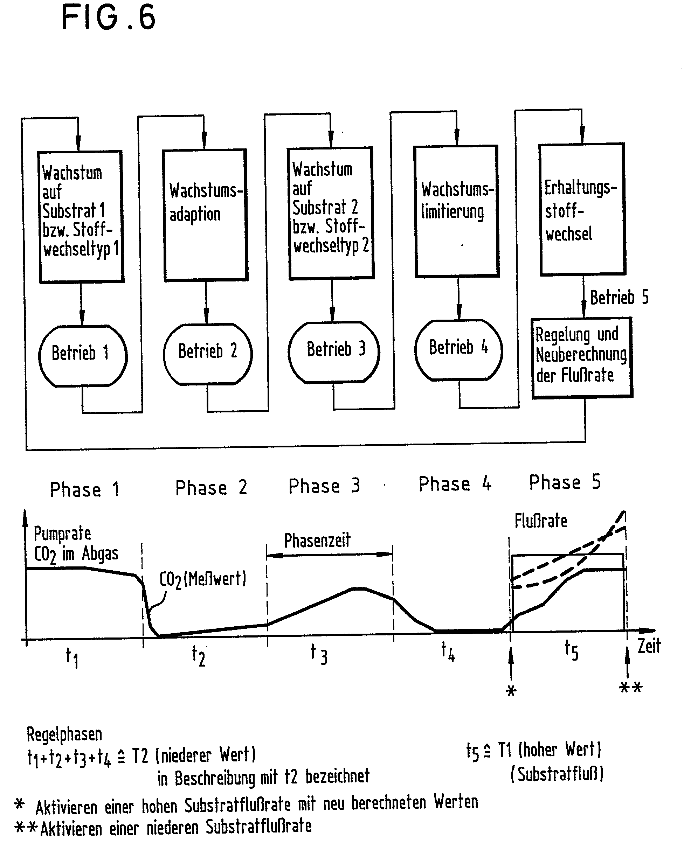

Jeder wichtigen Wachstumsphase der Kultur ist im Prinzip eine Ablaufphase (t1 bis t5, Fig. 6) der Regelung zugeordnet. Zur Vereinfachung der Regelungsstruktur können mehrere Wachstumsphasen auch durch eine einzige Ablaufphase der Regelung repräsentiert werden. Umgekehrt können einer Wachstumsphase zur Vereinfachung der Auswertung der Meßgrößen, zur Erhöhung der Betriebssicherheit der Regelung oder zur Verbesserung des Regelungsverhaltens auch mehrere aufeinanderfolgende Ablaufphasen der Regelung zugeordnet werden.

Da die verschiedenen Wachstumsphasen sich durch die Stoffwechselaktivität und die jeweils ablaufenden Reaktionen unterscheiden, sind den Wachstumsphasen auch typische Verläufe der Meßgrößen zugeordnet. Die Wachstumsphasen können daher prinzipiell auch über die Meßgrößen erkannt und beschrieben werden.

Typische Beispiele aus der Hefeproduktion wären z.B.

Definition "oxidativer Stoffwechsel" auf Glukosesubstrat = Wachstumsphase "starke Glukoselimitierung":

Die spezifische Sauerstoffaufnahmerate ist hoch und gleich der spezifischen Kohlendioxidproduktion; der Respirationsquotient = 1.

Definition "oxidativer Stoffwechsel" auf Ethanolsubstrat = Wachstumsphase "Ethanolwachstum":

Die Spezifische Sauerstoffaufnahmerate ist hoch; die spezifische Kohlendioxidproduktion ist niedrig; der

4

Respirationsquotient ist kleiner als 1.

Definition "fermentativer Stoffwechsel" auf Glukosesubstrat = Wachstumsphase "Glukoseüberschuß": Die spezifische Sauerstoffaufnahmerate ist niedrig; die spezifische Kohlendioxidproduktion ist hoch; der Respirationsquotient ist größer als 1.

Bei der erfindungsgemäßen Regelung werden die Wachstumsphasen zur Festlegung der Ablaufphasen der Regelung jedoch nicht unmittelbar über die Meßgrößen erkannt, sondern über deren dynamischen Verlauf.

Mittels eines logischen Modelles des Prozesses erkennt die Regelung den Übergang von einer Wachstumsphase zur nächsten, wobei durch den Übergang von einer Phase zur anderen die Phase selbst beschrieben wird. Das in der Regelungsstruktur verwendete Strukturmodell weist verschiedene definierte Modellphasenstrukturen auf, denen definierte Modellverläufe der normierten Meßgrößen zugeordnet sind.

Beim Regelprozeß werden die verschiedenen Modellstrukturen phasenweise hintereinander abgefragt und mit dem realen nomierten Verlauf der Meßgrößen verglichen. Durch diesen Vergleich werden dynamisch die Wachstumsphasen erkannt und damit die Ablaufphasen der Regelung festgelegt. Daraus errichnet die Regelungsanordnung, das Tastverhältnis, die Periodendauer und die Pumprate für den Folgezyklus. Sie bestimmt also den zeitlichen Verlauf der Pumprate PR (Fig. 2 bis 4). Die Pumprate PR, auch als momentane Flußrate bezeichnet, ist die pro Zeiteinheit zu einem bestimmten Zeitpunkt in den Fermenter gegebene Substratmenge, gemessen in entsprechenden physikalischen Einheiten, z.B. Liter pro Stunde oder Gramm pro Stunde. Ausgangsgröße (Stellgröße) der Regelung über der Zeit ist diese momentane Flußrate.

Es ist jedoch anzumerken, daß die Anzahl der zu verwendenden logischen Modellstrukturen/Ablaufphasen der Regelung wesentlich von der Komplexität des Wachstumsprozesses und dem Umfang der zusätzlichen Sicherheitsüberprüfungen abhängt. So kann es bei anderen als den beschriebenen Prozessen erforderlich sein, das logische Modell, die Anzahl der Regeln und damit die Anzahl der unterschiedlichen Wachstumsphasen zu erweitern oder zu reduzieren. Die Anzahl der Regeln ist somit kein fester Parameter des Verfahrens.

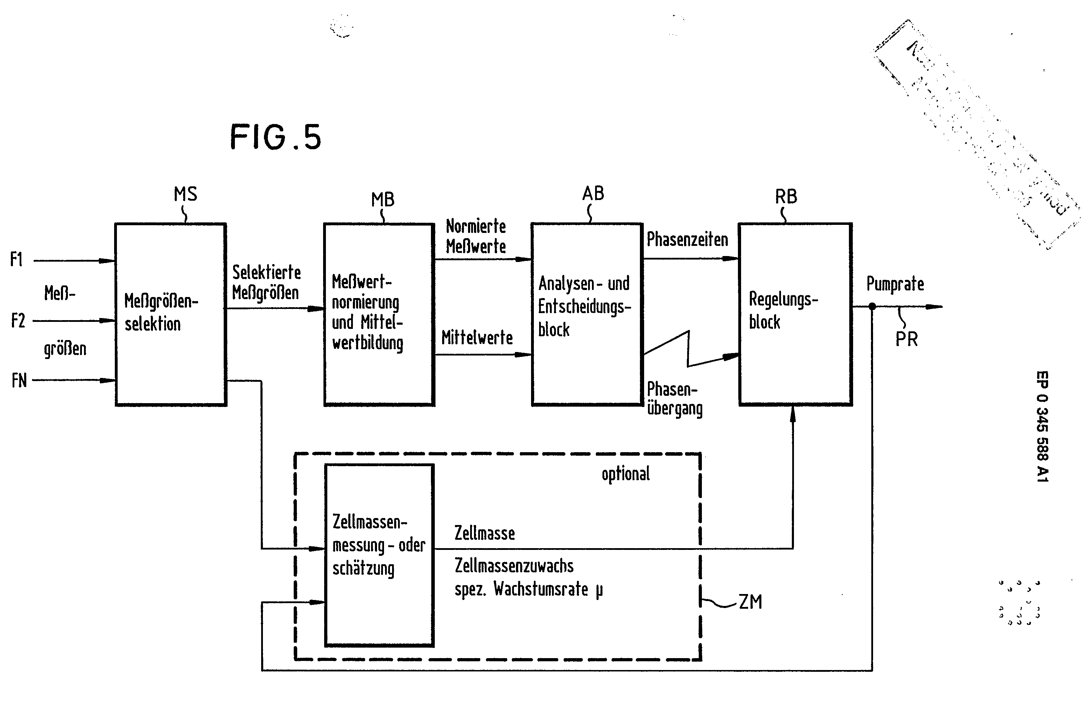

Die erfindungsgemäße Regelstruktur wird nun im folgenden anhand des Blockdiagrammes der Fig. 5 näher erläutert.

Abhängig von der Art des verwendeten Wachstumsprozesses können über einen Meßgrößenselektor MS die von Meßwertgebern gelieferte Meßgrößen selektiert werden. Die selektierten Meßgrößen werden in einem Meßwertanalyse- und Vorverarbeitungsblock MB normiert und es erfolgt eine Mittelwertbildung. Diese normierten Meßwerte bzw. die gebildeten Mittelwerte werden einem Analysen- und Entscheidungsblock AB zugeführt. Der Analysen- und Entscheidungsblock erkennt mit Hilfe einer logischen Modellstruktur den Übergang von einer Wachstums phase zu der nächsten und bestimmt damit die Phasenzeiten, bzw. die Phasendauer eines Zyklus. Die Phasenzeit ist dabei die Zeitdauer einer Ablaufphase der Regelung und hat damit auch die Dauer der von den Ablaufphasen repräsentierten Wachstumsphasen oder Gruppe von Wachstumsphasen, da den Ablaufphasen der Regelung entsprechende Wachstumsphasen zugeordnet sind.

Die aus dem Übergang von einer Wachstumsphase zur nächsten mittels des logischen Modelles ermittelten Phasenzeiten werden einem Regelungsblock RB zugeführt.

Der Regelungsblock RB errechnet aus den Phasenzeiten die neu einzustellenden Werte der Pumprate PR der Substratpumpe P (Pos. 1).

Diese Substratpumpe P bildet das Stellglied der Regelung. Anstelle der Pumpe ist jedoch auch jede andere Vorrichtung denkbar, die die Flußrate beeinflußt. Der Zusammenhand zwischen Ansteuersignal des Stellgliedes, das dem Ausgangssignal der Regelung entspricht und dem Ausgangssignal des Stellgliedes ist durch die Kennlinie des Stellgliedes gegeben. Falls sehr kurze Substratimpulse wünschenswert sind, z.B. beim Repeated-Batch, kann auch ein diskontinuierliches Stellglied verwendet werden, bei dem z.B. durch Entleeren eines Vorratsgefäßes plötzlich eine bestimmte Menge Substrat in den Reaktor BR gegeben wird.

Zur Ermittlung der Zellmasse und der spezifischen Wachstumsrate $\mu$, d.h. dem Zellmassenzuwachs, kann zwischen dem Regelungsblock RB und dem Meßgrößenselektor MS eine Zellmassenmeß- oder -schätzeinrichtung ZM angeordnet sein.

Diese Zellmassenmeß- oder -schätzeinrichtung ZM berücksichtigt die selektierten Meßgrößen des Meßgrößensektors MS und führt die errechneten und ermittelten Werte über die Zellmasse und den Zellmassenzuwachs $\mu$ dem Regelungsblock RB zu.

Die Zellmassenmeß- oder -schätzeinrichtung ZM berücksichtigt beim Berechnungs- und Ermittlungsvorgang auch die Pumprate PR bzw. die Flußrate.

Im folgenden werden nun die einzelnen Blöcke des Regelsystems näher beschrieben.

Analysen- und Entscheidungsblock.

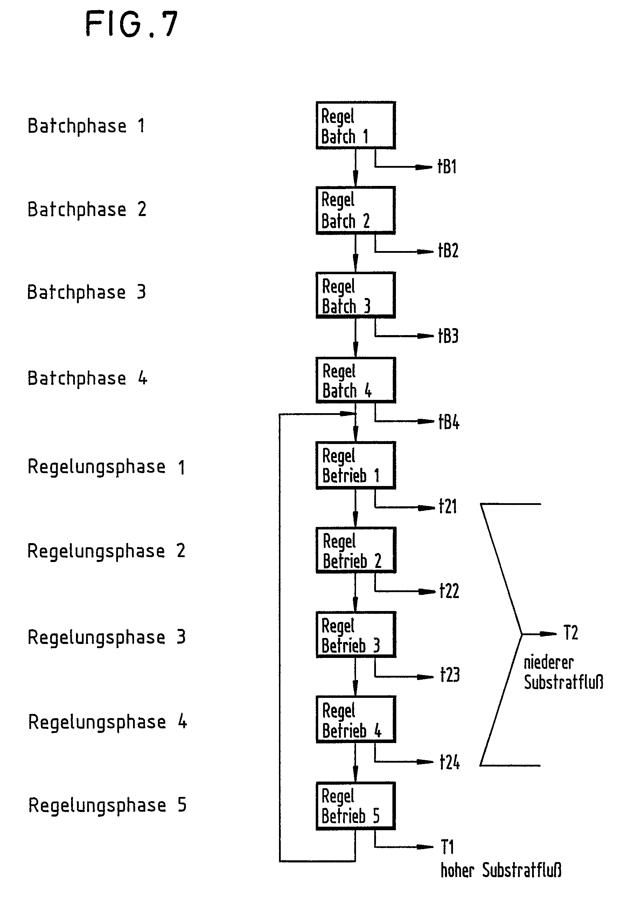

Der als mikroprozessorgesteuerte Rechner ausgebildete Analysen- und Entscheidungsblock AB enthält logische Strukturen in Form von logischen Regeln, die logische Modelle des Prozeßverlaufes in verschiedenen Wachstumsphasen des Prozesses repräsentieren. Mittels dieser logischen Modelle erkennt die Regelung den Übergang von einer Wachstumsphase zur nächsten. Hierbei wird gemäß Fig. 7 im Prinzip unterschieden zwischen logischen Regeln bzw. logischen Strukturen für den anlauf des Prozesses (Batch-Regel) und Regeln bzw. logische Strukturen für den geregelten, d.h. eingeschwungenen Betrieb (Betrieb 1 5).

Das in der Fig. 6 dargestellte vereinfachte Diagramm des Analysen- und Entscheidungsblockes AB enthält dabei nur die logischen Strukturen für den eingeschwungenen Betrieb. Ausführlicher sind die Strukturen in der Fig. 7 dargestellt.

Das logische Modell besteht beispielsweise aus 4 logischen Strukturen Batch 1 bis Batch 4 für den Anlauf des Prozesses und 5 logischen Strukturen Betrieb 1 bis Betrieb 5 für den eingeschwungenen Zustand. Die Anzahl der logischen Strukturen ist jedoch von der Art des Prozesses abhängig. Die den logischen Strukturen zugrunde liegenden Regeln werden später ausführlicher beschrieben. Im Prinzip handelt es sich bei den logischen Strukturen um Software-Strukturen, die bestimmte Wachstumsphasen des biologischen Wachstumsprozesses modellmäßig beschreiben. Die logischen Strukturen Batch 1 bis Batch 4 bzw. Betrieb 1 bis Betrieb 5, werden in einem Zyklus hintereinander abgefragt und so die einzelnen Phasenzeiten der Wachstumsphasen und damit die Dauer der Regelphasen ermittelt.

Im Gegensatz zur Beschreibung der Wachstumsphasen über die Werte der Meßgrößen definieren diese logischen Regeln des logischen Modells den Übergang von einer Regelungsphase zur nächsten und definieren damit auch die Regelungsphase selbst. Der Analyser- und Entscheidungsblock AB erkennt so den Übergang von einer Wachstumsphase zur folgenden.

Im folgenden werden die den einzelnen logischen Strukturen Batch 1 bis Batch 4 bzw. Betrieb 1 bis Betrieb 5 zugrunde liegenden logischen Zuordnungsvorschriften kurz als Regeln bezeichnet, näher beschrieben.

Die Strukturen Batch 1 bis Batch 4 ermitteln die Phasenzeiten tB1 bis tB4 für den Batch-Prozeß, d.h. für den Prozeßanlauf. Die Strukturen Betrieb 1 bis Betrieb 4 ermitteln die Phasenzeiten t21 bist t24 bei geregeltem Betrieb. Die Dauer der Regelphase 5 t1 ist die Zeit, in der der Substratfluß hohe Werte annimmt (Fig. 2). Die Summe der Regelungsphasen 1 bis 4, d.h. die Summe der Phasenzeiten t21 bis t24, ist die Zeit T2, in der der Substratfluß niedrige Werte annimmt.

Der zeitliche Ablauf der Regelung wird also durch den Analysen- und Entscheidungsblock AB gesteuert und zwar dadurch, daß dieser die Zeitpunkte erkennt, bei denen die Pumprate PR von niedrigen auf hohe Werte verändert werden muß und umgekehrt.

Im dargestellten Fall greift die Regelung zweimal während eines Zyklus Z ein. Nach Durchlauf der Phasen 1 bis Phasen 4 wird zu Beginn der Phase 5 eine Substratflußrate mit entsprechenden neu berechneten Werten auf Basis der Phasenzeiten t21 bis t24 aktiviert. Nach Durchlauf der Phase 5, d.h. nach Durchlauf der Zeit T1 (Fig. 2), wird am Ende dieser Regelungsphase 5 eine niedrige Substratflußrate ausgelöst.

Es ist jedoch darauf hinzuweisen, daß der Analysen- und Entscheidungsblock AB beim Dedektieren einer Alarmbedingung auch während eines Zyklus erneut eingreifen und die Pumprate verändern kann.

In den angegebenen Regeln sind die Konstanten IN1MIB, IN2MIB, IN1INC, IN1MIN, IN2MIN, IN1LIM, IN2LIM, TIMLIM, TIMMIN und KTIM2 sowie BA1AKT bis BA4AKT und BE1AKT bis BE4AKT Designparameter der Regelung. Mit ihnen kann die Regelung an das spezielle Muster der Meßgrößen des jeweiligen Prozesses angepaßt werden.

Der Übergang von Batch- auf Fed-Batch- oder kontinuierlichen Betrieb (Chemostat) erfolgt durch erstmaliges Einschalten des Substratflusses genau dann, wenn die Substratlimitierung einsetzt. Dieser Übergang entspricht im Prinzip dem normalen Wiedereinschalten während der Pulszyklen und kann daher vom gleichen Analysen- und Entscheidungsblock erkannt werden, der für den Pulsbetrieb benutzt wird. In der praktischen Realisierung ist es unter Umständen günstiger, das erstmalige Einschalten durch einen eigenen Analysen- und Entscheidungsblock vornehmen zu lassen. Dieser kann dann spezielle logische Strukturen enthalten, die eine sichere Verfolgung des Batch-Laufes ermöglichen, z.B. die Regeln Batch 1 bis Batch 4 (Fig. 7).

Die Prozeßende-Bedingung kann das Erreichen eines bestimmten Reaktorvolumens, das Erreichen einer Flußrate oder eine bestimmte Reaktorkonzentration sein. Auch eine einfache Zeitbedingung ist als Definition des Prozeßendes möglich.

Für den automatischen Übergang von Wachstumsphase auf Produktionsphase lassen sich keine allgemeinen Kriterien angeben. Diese hängt stark vom eingesetzten Mikroorganismus ab. Der Analysen- und Entscheidungsblock stellt jedoch die Informationen zur Verfügung, die eine Automatisierung dieses Vorganges ermöglichen. Das logische Modell des Prozesses muß dann jedoch entsprechend durch zusätzliche Regeln erweitert werden. Beispielsweise kann der Übergang über eine einfache Zeitbedingung oder durch Erreichen einer bestimmten Konzentration im Fermenter definiert werden. Im Einzelfall, z.B. bei dem Hefeprozeß, kann die Wachstumsphase und die Produktionsphase auch mit Batch- und Fed-Batch-Betrieb identisch sein.

Regeln für den Start des Prozesses als Batch:

Batch-Phase 1: Wachstum auf erstem Substrat

Regel Batch 1: Übergang von Wachstum auf erstem Substrat zur Lagphase.

Bestimme Anfangszeit der Regelungsphase.

Falls BA1AKT gleich Null ist,

setze tB1 = 0 und aktiviere Regel Batch 2.

Sonst warte bis der normierte Meßwert 1 auf IN1MIB abfällt,

oder der Substratfluß manuell eingeschaltet wurde.

Dann bestimme tB1 = aktuelle Zeit - Anfangszeit und gehe zu Regel Batch 2.

Batch-Phase 2: Lagphase

Regel Batch 2: Übergang von der Lagphase zum Wachstum auf zweitem Substrat.

Bestimme Anfangszeit der Regelungsphase.

Falls BA2AKT gleich Null ist,

setze tB2 = 0 und aktiviere Regel Batch 3.

Sonst warte bis aktuelle Zeit - Anfangszeit größer als KTIM2 x tB1 ist,

oder der normierte Meßwert 2 auf IN2MIB abfällt,

oder der Substratfluß manuell eingeschaltet wurde.

Dann bestimme tB2 = aktuelle Zeit - Anfangszeit und gehe zu Regel Batch 3.

Batch-Phase 3: Wachstum auf zweiten Substrat

Regel Batch 3: Übergang von Wachstum auf zweitem Substrat zum Erhaltungsstoffwechsel bzw. zur Limitierung.

Bestimme Anfangszeit der Regelungsphase.

Falls BA3AKT gleich Null ist,

setze tB3 = 0 und aktiviere Regel Batch 4.

Sonst warte bis der auf den gleitenden Mittelwert bezogene normierte Meßwert 1 auf IN1INC ansteigt,

oder der normierte Meßwert 1 auf IN1MIN abfällt,

oder der Substratfluß manuell eingeschaltet wurde.

Dann bestimme tB3 = aktuelle Zeit - Anfangszeit und gehe zu Regel Batch 4.

Batch-Phase 4: Limitierung

Regel-Batch 4: Übergang vom Batch-Betrieb auf Regelungsbetrieb, Sicherheitsabfragen und Übergangsbedingungen.

Falls die Prozeßendebedingung erreicht ist beende den Prozeß Sonst bestimme Anfangszeit der Regelungsphase.

Warte bis aktuelle Zeit - Anfangszeit größer als TIMLIM ist.

Dann, falls BA4AKT gleich Null ist, setze tB4 = 0 und aktiviere Regelungs-Phase 5.

Sonst warte bis der normierte Meßwert 2 auf IN2MIN abfällt, oder der Substratfluß manuell eingeschaltet wurde.

Dann bestimme tB4 = aktuelle Zeit - Anfangszeit und gehe zu Regelungs-Phase 5.

Regeln für den automatisierten (geregelten) Betrieb des Prozesses als Repeated-Batch, Fed-Batch oder Chemostat:

Regelungs-Phase 1: Wachstum auf erstem Substrat (Substratüberschuß)

Regel Betrieb 1: Übergang von Wachstum auf erstem Substrat zur Lagphase.

Bestimme Anfangszeit der Regelungsphase.

Falls BE1AKT gleich Null ist,

setze t21 = 0 und aktiviere Regelungsphase 2.

Sonst warte bis der normierte Meßwert 1 auf IN1MIN abfällt,

oder der Substratfluß manuell eingeschaltet wurde.

Dann bestimme t21 = aktuelle Zeit - Anfangszeit und gehe zu Regelungs-Phase 2.

Regelungs-Phase 2: Lagphase

Regel Betrieb 2: Übergang von der Lagphase zum Wachstum auf zweitem Substrat.

Bestimme Anfangszeit der Regelungsphase.

Falls BE2AKT gleich Null ist,

setze t22 = 0 und aktiviere Regelungs-Phase 3.

Sonst warte bis aktuelle Zeit - Anfangszeit größer als t2.TIMMIN ist,

oder der Substratfluß manuell eingeschaltet wurde.

Dann bestimme t22 = aktuelle Zeit - Anfangszeit und gehe zu Regelungs-Phase 3.

Regelungsphase 3: Wachstum auf zweitem Substrat.

Regel Betrieb 3: Übergang vom Wachstum auf zweitem Substrat zum Erhaltungsstoffwechsel.

Bestimme Anfangszeit der Regelungsphase.

Falls BE3AKT gleich Null ist,

setzte t23 = 0 und aktiviere Regelungs-Phase 4.

Sonst warte bis der auf den gleitenden Mittelwert bezogene normierte Meßwert 1 auf IN1INC ansteigt,

oder der auf den gleitenden Mittelwert bezogene normierte Meßwert 1 auf IN1LIA abfällt,

oder der Substratfluß manuell eingeschaltet wurde.

Dann bestimme t23 = aktuelle Zeit - Anfangszeit und gehe zu Regelungs-Phase 4.

Regelungsphase 4: Limitierung

Regel Betrieb 4: Übergang von der Limitierung zum Wachstum auf erstem Substrat. Einschalten eines hohen Substratflusses.

Bestimme Anfangszeit der Regelungsphase.

Falls BE4AKT gleich Null ist,

setze t24 = 0 und aktiviere Regelungs-Phase 5.

Sonst warte bis der normierte Meßwert 1 auf IN1LIM abfällt,

oder der normierte Meßwert 2 auf IN2LIM ansteigt,

und/oder aktuelle - Anfangszeit größer als TIMLIM ist,

oder der Substratfluß manuell eingeschaltet wurde.

Dann bestimme t24 = aktuelle Zeit - Anfangszeit und gehe zu Regelungs-Phase 5.

Regelungs-Phase 5: Überwachung der Einschaltphase des Substratflusses und des Erreichens der Wachstumsphase auf dem ersten Substrat.

Regel Betrieb 5: Übergang von Limitierung auf Wachstum.

Falls die Prozeßendebedingung erfüllt ist, beende den Prozeß.

Sonst bestimme T2 = t21 + t22 + t23 + t24 und bestimme die Anfangszeit der Regelungsphase.

Dann warte bis der normierte Meßwert 1 auf IN1MIN ansteigt und der normierte Meßwert 2 auf IN2LIM abfällt,

oder der Substratfluß eine Zeitdauer t11 eingeschaltet war, oder die Phase manuell beendet wurde.

Falls die Meßwertbedingungen nicht erfüllt worden sind und die Phase nicht manuell beendet worden ist, gib Alarm, oder erhöhe Substratfluß,

oder wechsle Meßwertkanal (Sensor),

und dann wiederhole die Regelungs-Phase 5.

Sonst bestimme T1 = aktuelle Zeit - Anfangszeit und gehe zu Regelungs-Phase 1.

Regelungsblock.

Der als mikroprozessorgesteuerte Rechnereinheit ausgebildete Regelungsblock RB weist eine erste logische Struktur L1 auf, die dazu dient, dem Pumpratenstartwert bei Beginn eines jeden Zyklus (Phase 5) zu errechnen. Zu diesem Zwecke werden dem logischen Block L1 die vom Analysen- und Entscheidungsblock AB ermittelten Phasenzeiten zugeführt. Weiterhin berücksichtigt er Sollwerte oder Sollwertprofile für Phasenzeiten und Tastverhältnis.

Weitere logische Strukturen L2 und L3 dienen der Ermittlung des Pumpratenprofiles aus den vom Analysen- und Entscheidungsblock ermittelten Phasenwechsel. Diese Phasenwechsel sind der Übergang von der Phase 4 zur Phase 5, mit Aktivierung einer hohen Substratflußrate mit neu berechneten Werten und der Übergang von Phase 5 auf Phase 4 mit Aktivierung einer niederen Substratflußrate. Die logischen Strukturen L2 und L3 wählen also ensprechend den Darstellungen der Figuren 2 bis 4 die Impulsform der Pumprate PR aus.

Die Auswahl der Impulsform orientiert sich an der Dauer derPulszyklen, am eingesetzten Mikroorganismus und am momentanen Betriebszustand des Prozesses. Der Verlauf der Pulse sollte an den sich zeitlich verändernden Substratbedarf bzw. an die Wachstumsrate der Mikoorganismen angepaßt sein. Bei kurzen Pulszyklen (Minutenbereich) können jedoch zur Vereinfachung des Verfahrens generell Rechteckimpulse verwendet werden. Bei exponentiellem Wachstum der Kultur sind allgemein Impulse der Form (0 <T-T0 < T1-T0).
$$Pexp(T-T0) = a.e^{b.(T-T0)}$$
und bei linearem Wachstum (z.B. Sauerstofflimitierung)
$$Plin(T-T0) = c.(T-T0) + d$$

angezeigt. Die Parameter a und d stellen die Pulshöhe beim Einschalten des Substratflusses dar. Diese wird durch den Regelungsblock bestimmt. Die Parameter b und c legen den Zuwachs des Substratflusses innerhalb des Zyklus fest. Sie können zur Vereinfachung konstant gehalten werden. Besser ist jedoch eine automatische Anpassung an die Wachstumsgeschwindigkeit der Kultur. Die spezifische Wachstumsrate $\mu$ der Kultur kann über direkte Messung der Zellkonzentration, mittels mathematischer Modelle oder indirekt aus der Flußrate selbst berechnet werden. Im letzteren Fall gilt unter der Vereinfachung, daß die Stoichiometrie des Wachstums konstant bleibt:

$$\mu = \frac{\text{mittlere Flußrate . Substratreservoirkonzentration}}{\text{Mediumvolumen . Zellkonzentration . Ausbeute}}$$

Daraus folgt für die Parameter b und c:

$$b = \mu$$

$$c = d . \frac{e^{\mu(T1+T2)}-1}{T1 + T2}$$

Eine Verbesserung des Verfahrens kann dadurch erreicht werden, daß man basierend auf der zeitlichen Tendenz von $\mu$ (betimmt aus den letzten berechneten oder bemessenen Werten) eine Vorausschätzung von $\mu$ für den nächsten Pulszyklus macht und diese zur Bestimmung von b und c heranzieht.

Entsprechende Pulsformen mit anderen Werten der Parameter a, b, c und d können in der Regelungsphase T1<T<T2 verwendet werden. Als Sonderfall kann jedoch auch a = b = c = d = 0 gesetzt werden, d.h. der Substratfluß ist ausgeschaltet.

Die Auswahl der Impulsform erfolgt momentan in der Designphase der Regelung. Eine automatische Auswahl, oder eine zeitlich gesteuerte Auswahl während des Prozesses ist jedoch realisierbar.

Im Detail ist im Regelungsblock RB folgender prinzipieller Ablauf realisiert:

Warte auf Erreichen der Regelungsphase 5, d.h. solange bis Regel Batch 4 oder Regel Betrieb 4 erfüllt ist.

Bestimme die neuen einzustellenden Werte der Pumprate aus den Phasenzeiten tB1 bis tB4 bzw. t21 bis t24 und T1.

Fahre ein Profil der Pumprate mit hoher Flußrate entsprechend der vorgegebenen Pulsform, solange·bis Regelbetrieb 5 erfüllt ist oder die Alarmbedingung von Regel Betrieb 5 eintrifft. In diesem Fall erhöhe die Flußrate und wiederhole die Regelungsphase.

Fahre ein Profil der Pumprate mit niedriger Flußrate entsprechend der vorgegebenen Pulsform, solange bis Regel Betrieb 4 erfüllt ist.

Wiederhole den gesamten Ablauf.

Der zeitliche Ablauf der Regelung wird wie bereits beschrieben durch den Analysen- und Entscheidungsblock derart gesteuert, daß dieser die Zeitpunkte erkennt, bei denen die Pumprate von niedrigen auf hohe Werte verändert werden muß und umgekehrt.

Berücksichtigt wird dabei auch die spezifische Wachstumsrate $\mu$ der Kultur bzw. die Zellmasse X.

Wie bereits eingangs ausgeführt, läßt sich die mittlere Flußrate, d.h. die über einen bestimmten Zeitpunkt ermittelte Pumprate in einem Zyklus über die Phasenzeiten T1 und T2, also über das Tastverhältnis und/oder über die Werte der Pumprate PR in den beiden Zeitabschnitten T1 und T2 einstellen.

Die Phasenzeiten sind dabie Ausgangsgrößen des Analysen-und Entscheidungsblockes AB. Die Pumprate PR ist Ausgangsgröße der Regelung in Abhängigkeit von den sich einstellenden Phasenzeiten T1 und t21 bis t24.

Die Regelung kann dadurch vereinfacht werden, daß sie die Phasenzeiten t21 bis t21 nicht getrennt berücksichtigt, sondern nur deren Summe T2 (Fig. 7). Folgende Betriebsarten sind dann als spezielle Realisierung der Regelung möglich:

Die mittlere Flußrate pro Zyklus wird über Variation der Pumprate und der Zeit T1 eingestellt. Die Zeit T2 ergibt sich aus dem Analysen- und Entscheidungsblock. Regelgröße des Systems ist das Tastverhältnis oder die Zeit T2. Die entsprechenden Sollwerte sind konstant oder folgen einem vorgegebenen Verlauf.

Die Flußrate wird nur über Variation der Pumprate PR eingestellt. Die Zeit T1 bleibt konstant. Die Zeit T2 ergibt sich aus dem Analysen- und Entscheidungsblock. Regelgröße des Systems ist das Tastverhältnis oder die Zeit T2. Die entsprechenden Sollwerte sind konstant oder folgen einem vorgegebenen Verlauf.

Die mittlere Flußrate wird nur über Variation der Zeit T1 eingestellt. Die Pumprate PR bleibt auf einem voreingestellten Pulsverlauf. Die Zeit T2 ergibt sich aus dem Analysen-und Entscheidungsblock AB. Regelgröße des Systems ist das Tastverhältnis. Der entsprechende Sollwert ist konstant oder folgt einem vorgegebenen Verlauf.

Die Regelungseigenschaften lassen sich durch folgende Designparameter an die unterschiedlichsten Erfordernisse anpassen:
Die Regelverstärkung beeinflußt die Stabilität und das Einschwenkverhalten; das Tastverhältnis, die Gleichförmigkeit der Betriebsbedingungen; die Periodendauer die Betriebsart von direkter Regelung bis Steuerung mit wiederholter Adaption; die Pulsform die Abstimmung auf das biologische System und die Betriebsart; und die Phasenakativierung/Inaktivierung die Anpassung an unterschiedliche Mikroorganismen und Produkte.

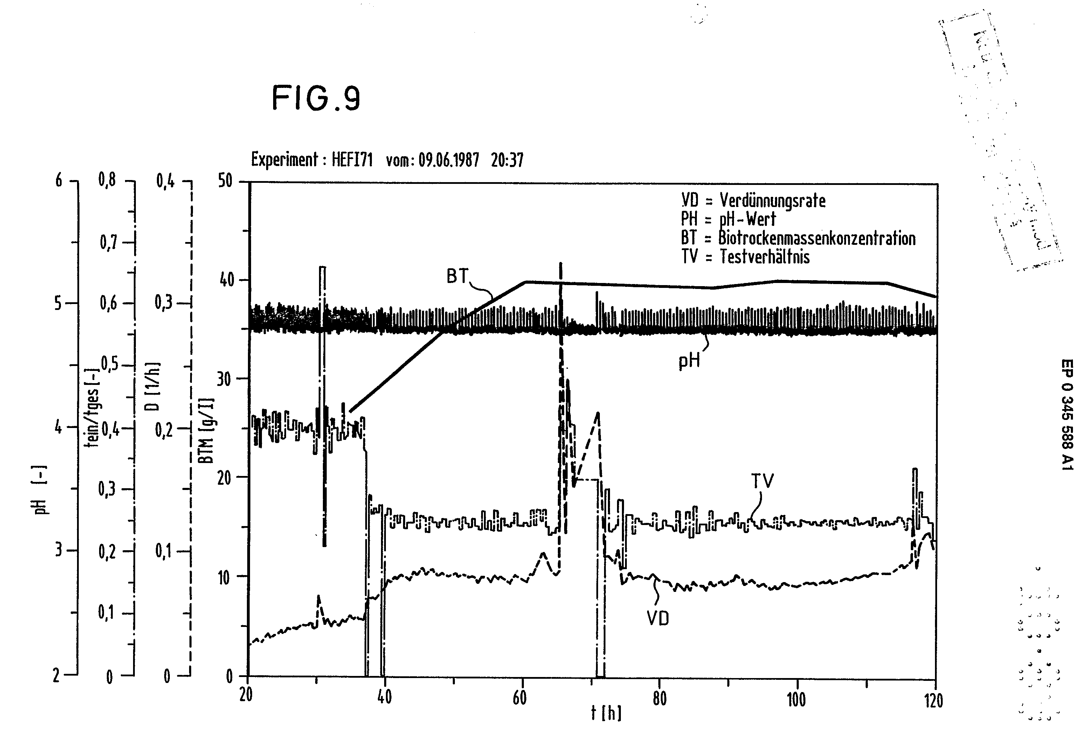

Im folgenden sollen nun anhand der Fig. 9 bis 13 Anwendungsbeispiele des erfindungsgemäßen Systems näher beschrieben werden.

Beispiel 1: Hefekultivierung im Chemostaten.

Bei der anaeroben, kontinuierlichen Alkoholproduktion müssen ständig alte Zellen regeneriert oder durch neue ersetzt werden. Man benötigt dazu einen zweistufigen Prozeß, bei dem Zellen kurzfristig aus der Alkoholproduktionsstufe entnommen und in einer aeroben Wachstumsstufe regeneriert werden, oder man führt ständig frische Zellen aus einer vorgeschalteten Zellproduktionsstufe zu. Bei letzterem Weg sind eine hohe Zellkonzentration, eine hohe Zellproduktivität und ein intakter Enzymhaushalt der Zellen wünschenswert. Diese Forderungen konnten mit dem erfindungsgemäßen System durch Regelung der Verdünnungsrate erfüllt werden. Als Meßgröße diente dabei der Ph-Wert des Mediums. Fig. 9 zeigt die experimentellen Ergebnisse für einen Zeitabschnitt der Chemostatkultivierung, den Ph-Wert PH, die Biotrockenmassenkonzentration PT, die Verdünnungsrate VD und das Tastverhältnis TV der gepulsten Substratzufuhr. Nach der anfänglichen instationären Wachstumsphase mit steigenden Zellkonzentrationen ermöglicht die Regelung einen stabilen Betrieb ohne Gefahr des Auswaschens bei hohen Verdünnungsraten und Zellkonzentrationen, die sonst nur durch eine aufwendige Zellrückführung erreicht werden können.

Beispiel 2: Backhefekultivierung im Fed-Batch.

Eine optimale Regelung des Substratzulaufes bei der Backhefefermentation muß den Stoffwechsel in der Nähe des kritischen Umschaltpunktes von oxidativen zu fermentativen Wachstum halten. Dazu ist üblicherweise ein hoher Aufwand erforderlich, der den praktischen industriellen Einsatz solcher Regelungsstrategien entgegensteht: On-line-Messung der Äthanolkonzentration, genaue Bilanzierung der Abgaswerte zur Berechnung des Respirationsquotienten, oder Benutzung modell gestützter Verfahren.

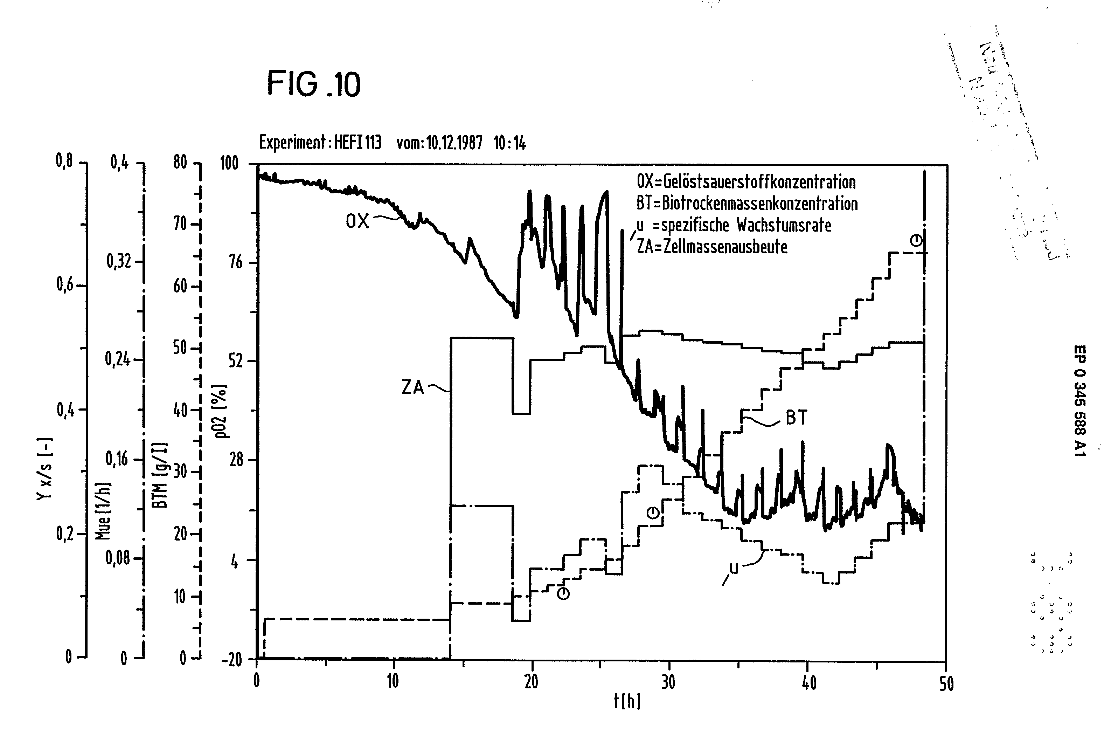

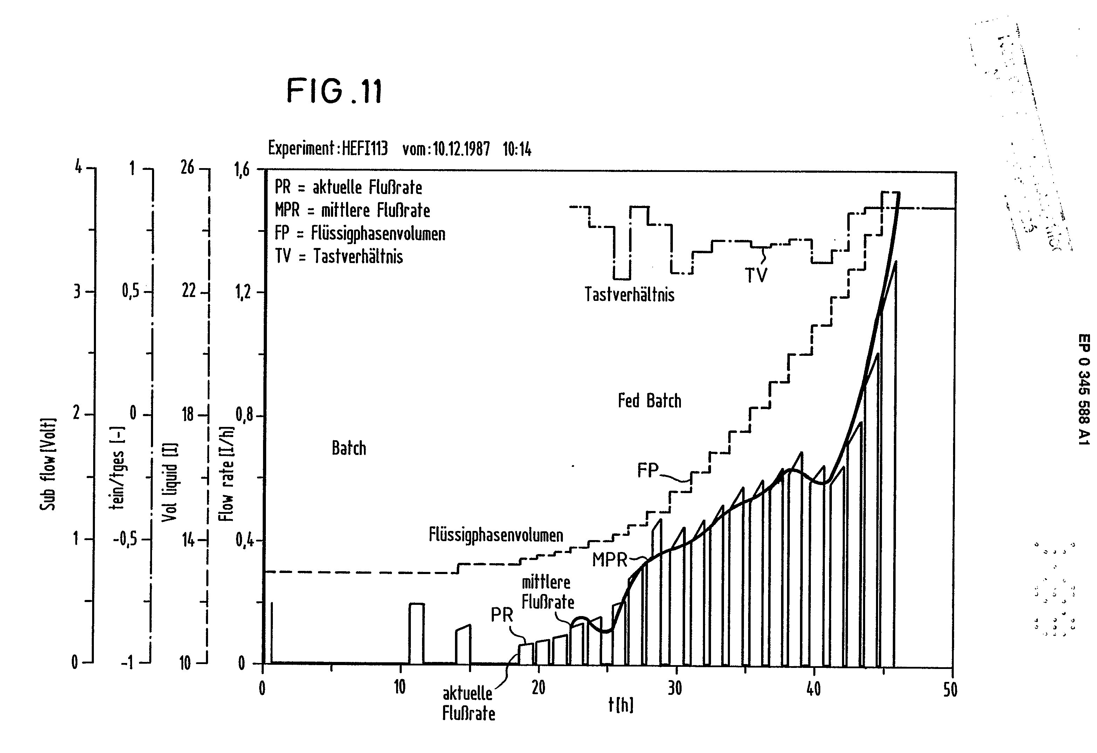

Mit dem erfindungsgemäßen System kann eine derartige Regelung verwirklicht werden, die nur die Messung von pH oder der Kohlendioxydkonzentration im Abgas benötigt. Da der Hefestoffwechsel sehr komplex ist, müssen alle fünf Regelungsphasen während eines Zyklus berücksichtigt werden. In den Fig. 10 und 11 sind die experimentell erhaltenen Ergebnisse dargestellt. Fig. 10 gibt neben der gelöst Sauerstoffkonzentration OX auch die Ergebnisse einer On-line-Schätzung der Biotrockenmassenkonzentration BT, der spezifischen Wachstumsrate $\mu$ und der Zellmassenausbeute ZA wieder. Es konnte eine sehr gute Übereinstimmung mit den Meßwerten erzielt werden. Fig. 11 zeigt den Verlauf der aktuellen und mittleren Flußrate (PR, MPR), das Flüssigphasenvolumen FP und des Tastverhältnisses TV.

Der Prozeß wird als Batch gestartet. Das System erkennt das Ende des Batch-Abschnittes und startet den Fed-Batch Betrieb, wobei er sich selbständig an die momentane Wachstumsaktivität der Kultur anpaßt. Das Wachstum ist zunächst exponentiell und geht dann aufgrund der einsetzenden Sauerstofflimitierung in einen linearen Bereich über. Trotzdem kann durch die Regelung im gesamten Verlauf eine hohe Ausbeute und Produktivität erreicht und bis zu sehr hohen Zellkonzentrationen aufrecht erhalten werden, ohne daß sich Ethanol anreichert.

EP 0 345 588 A1

Der Pulsbetrieb bewirkt einen ständigen Wechsel zwischen leicht fermentativen und vollständigem oxidativem Wachstum, wobei auch Äthanol als Substrat dient. Durch geeignete Veränderung des Tastverhältnisses und der Periodendauer besteht zusätzlich die Möglichkeit, die Eigenschaften der Hefezelle (z.B. Proteingehalt) in der gewünschten Weise zu beeinflussen.

Beispiel 3: Fed-Batch-Produktion von 2,3 Butandiol.

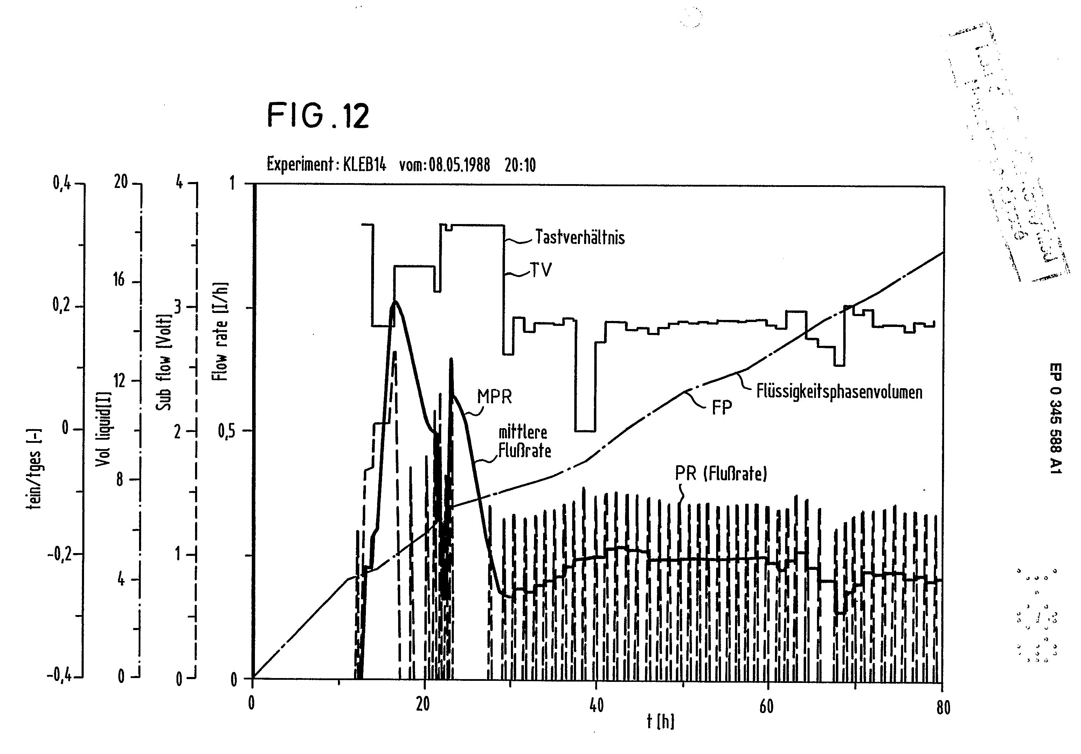

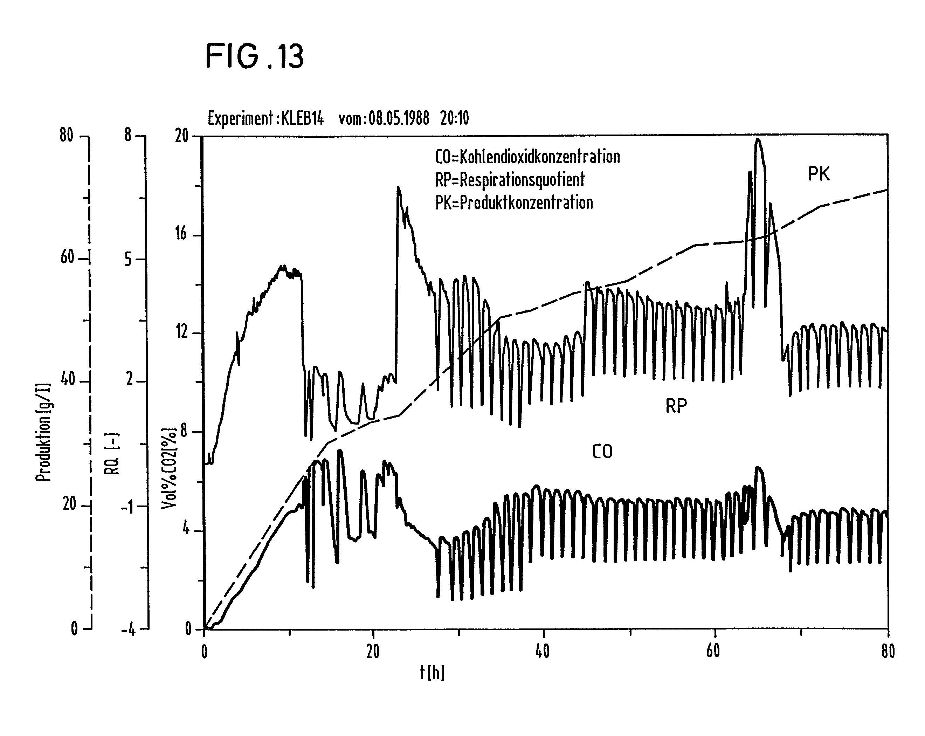

Im Rahmen der Gewinnung hochwertiger chemischer Substanzen aus landwirtschaftlichen Abfallproduktien und natürlichen Rohstoffen ist die Produktion von Butandiol ein interessanter Prozeß. Da hohe Substratkonzentrationen das Wachstum und das Produkt inhibieren, ist auch hier eine Prozeßführung mit wiederholter Substratzufuhr oder als Fed-Batch angezeigt. Weiterhin kann eine hohe Produktivität nur unter mikroaeroben Bedingungen erreicht werden. Die Fig. 12 zeigt die experimentellen Ergebnisse für die Flußrate (PR, MPR), das Flüssigphasenvolumen FP und das Tastverhältnis TV eines erfindungsgemäß geregelten Fed-Batch-Prozesses. Bei diesem Organismus braucht die Regelung die Wachstumsphase 2 nicht zu berücksichtigen. Die Substratversorgung der Kultur folgt automatisch dem aktuellen Bedarf, der durch den Grad der Sauerstofflimitierung und die fermentative Aktivität der Mikroorganismen bestimmt ist. Als Meßgröße für die Regelung dient hier die Kohlendioxydkonzentration CO im Abgas. Diese ist neben dem Respirationsquotienten R und der Produktkonzentration PK in Fig. 13 dargestellt. Es wurden Lösungsmittelkonzentrationen von mehr als 70 g je Liter erreicht.

Bezugszeichenliste

BR Bioreaktor
MO Mikroorganismus
M Rührwerk
P Substratpumpe
V Vorratsbehälter
MP Mediumpumpe
F1 Meßwertgeber Meßgröße 1
1 Meßgröße 1
F2 Meßwertgeber Meßgröße 2
2 Meßgröße 2
R Regelanordnung
Z Zyklus
T1 Zeitabschnitt mit hohem Substratfluß
T2 Zeitabschnitt mit niederem Substratfluß
t1-t5 Ablaufphasen der Regelung (Wachstumsphasen)
PR Pumprate (aktuelle Flußrate)
MS Meßgrößenselektor
MB Meßwertanalyse- und Vorverarbeitungsblock
AB Analysen- und Entscheidungsblock
RB Regelungsblock
ZM Zellmassenmeßeinrichtung
$\mu$ spezifische Wachstumsrate
x Biomasse
BATCH1-BATCH5 logische Strukturen für den Prozeßanlauf
Betrieb 1-Betrieb 5 logische Strukturen für den eingeschwungenen Betrieb
tB1-tB4 Phasenzeiten Batch
t21-t24 Phasenzeiten im eingeschwungenen Betrieb
Phase 1 - Phase 5 Regulungsphasen L1-L3 logische Strukturen Regelungsblock
PH ph-Wert
BT Biotrockenmassenkonzentration
VD Verdünnungsrate
TV Tastverhältnis
OX Gelöstsauerstoffkonzentration

11

ZA Zellmassenausbeute
MPR mittlere Flußrate
FP Flüssigphasenvolumen
CO Kohlendioxydkonzentration
RP Respirationsquotient
PK Produktkonzentration

## Ansprüche

1. Verfahren zur Regelung eines biologischen Wachstumsprozesses, bei dem einem in einem Reaktor (BR) befindlichen Mikroorganismus, wachstumsabhängig Substrat zugeführt wird, mit folgendem Merkmal:
- Die Zuführung des Substrates erfolgt in einer Folge von Zyklen (Z) bestehend aus Zeitabschnitten mit relativ hohem Substratfluß (T1) und Zeitabschnitten mit relativ niederem Substratfluß (T2).
- Mittels eines logischen Modelles (AB) des Wachstumsprozesses werden automatisch während eines Zyklus (Z) Wachstumsphasen des Mikroorganismus erkannt und in Abhängigkeit davon der Wachstumsprozeß geregelt.

2. Verfahren nach Anspruch 1, dadurch **gekennzeichnet,** daß die Substratzufuhr während der Regelung pulsweise erfolgt.

3. Verfahren nach einem der Ansprüche 1 oder 2, dadurch **gekennzeichnet,** daß die Wachstumsphasen über die Übergänge von einer Wachstumsphase zur anderen erkannt werden.

4. Verfahren nach Anspruch 2, dadurch **gekennzeichnet,** daß in Abhängigkeit von charakteristischen Größen des Wachstumsprozesses und/oder der Art des Mikroorganismus der Pulsverlauf der Substratzufuhr festgelegt wird.

5. Anordnung zur Regelung eines biologischen Wachstumsprozesses, bei dem einem in einem Reaktor (BR) befindlichen Mikroorganismus über eine Fördereinrichtung (P) Substrat zugeführt wird, dadurch **gekennzeichnet,** daß ein Regelkreis (R) vorgesehen ist, der mittels logischer Strukturen (AB) durch Vergleich eines Wachstumsprozeßmodelles mit dem tatsächlichen Verlauf des Wachstumsprozesses den Wachstumsprozeß regelt.

6. Anordnung nach Anspruch 5, dadurch **gekennzeichnet,** daß der Regelkreis (R) die während eines Zyklus (Z) auftretenden Wachstumsphasen des Mikroorganismus über logische Modellstrukturen (Batch 1 bis Batch 4, Betrieb 1 bis Betrieb 5) erfaßt und in Abhängigkeit davon den Verlauf der Substratzufuhr in einem Folgezyklus festlegt.

7. Anordnung nach einem der Ansprüche 5 oder 6, **gekennzeichnet** durch
- eine Fühleinrichtung (F1, F2, MS) zum Erfassen von prozeßrelevaten Meßgrößen,
- einen Analysen- und Entscheidungsblock (AB) zum Erfassen von charakteristischen Wachstumsphasen des Mikroorganismus über eine logische Modellstruktur während eines Zyklus (Z) und
- einen Regelungsblock (RB), der in Abhängigkeit von den ermittelten Wachstumsphasen über die Fördereinrichtung (P) die Substratzufuhr regelt.

8. Anordnung nach Anspruch 7, **gekennzeichnet** durch eine mit dem Regelungsblock (RB) bedarfsweise koppelbare Einrichtung (ZM), die Daten über die Zellmasse des Mikrorganismus (X) und/oder den spezifischen Zellmassenzuwachs ($\mu$) des Mikroorganismus ermittelt und dem Regelungsblock (RB) zuleitet.

9. Anordnung nach Anspruch 7, dadurch **gekennzeichnet,** daß der Analysen- und Entscheidungsblock (AB) eine Folge von den Wachstumsphasen (Phase 1 bis Phase 5) zugeordnete, entsprechend den verwendeten Wachstumsprozessen einstellbare Modellstrukturen (Batch 1 bis Batch 4, Betrieb 1 bis Betrieb 5) aufweist, die zur Erfassung der Wachstumsphasen hintereinander abfragbar sind und die Phasendauer (tB1 bis tB4, t21 bis t24, T1) der einzelnen Wachstumsphasen ermittelt.

10. Anordnung nach Anspruch 9, dadurch **gekennzeichnet,** daß der Analysen- und Entscheidungsblock (AB) eine erste (Batch) dem Prozeßanfahrvorgang zugeordnete und eine zweite (Betrieb) dem eingeschwungenen Prozeßbetrieb zugeordnete Folge von logischen Modellstrukturen aufweist.

11. Anordnung nach einem der Ansprüche 7 bis 10, dadurch **gekennzeichnet,** daß der Regelunsblock (RB) die Förderleistung (PR) der Fördereinrichtung (B) derart festlegt, daß das Substrat dem Reaktor (BR) in Zyklen (Z) pulsweise zugeführt wird, wobei
- erste logische Strukturen (L1) zur Bestimmung der Förderleistung (PR) der Fördereinrichtung bei Zyklusbeginn und

- zweite logische Strukturen (L2, L3) zur Bestimmung des zeitlichen Verlaufs der Förderleistung (PR) (Pumpenratenprofil) durch Auswahl der Impulsform vorgesehen sind.

# FIG.1

# FIG.2

Quasi-kontinuierlicher Fed-Batch oder Chemostat

Kurze Periodendauer
(Minutenbereich)

Rechteck-oder
Trapezimpulse

# FIG.3

Adaptiv gesteuerter Fed-Batch

Lange Periodendauer
(Stundenbereich)

Großes Tastverhältnis

Exponential-oder
Trapezimpulse

# FIG.4

Automatischer Repeated-Batch

Lange Periodendauer
(Stundenbereich)

Kleines Tastverhältnis

Rechteckimpulse

# FIG.5

**MS** — Meßgrößenselektion

**MB** — Meßwertnormierung und Mittelwertbildung

**AB** — Analysen- und Entscheidungsblock

**RB** — Regelungsblock

F1, F2, FN — Meßgrößen

Selektierte Meßgrößen

Normierte Meßwerte

Mittelwerte

Phasenzeiten

Phasenübergang

Pumprate — PR

optional

Zellmassenmessung - oder schätzung

Zellmasse

Zellmassenzuwachs spez. Wachstumsrate μ

**ZM**

EP 0 345 588 A1

# FIG.6

Wachstum auf Substrat 1 bzw. Stoff- wechseltyp 1

Wachstums- adaption

Wachstum auf Substrat 2 bzw. Stoff- wechseltyp 2

Wachstums- limitierung

Erhaltungs- stoff- wechsel

Betrieb 1

Betrieb 2

Betrieb 3

Betrieb 4

Betrieb 5

Regelung und Neuberechnung der Flußrate

Phase 1  Phase 2  Phase 3  Phase 4  Phase 5

Pumprate $CO_2$ im Abgas

$CO_2$ (Meßwert)

Phasenzeit

Flußrate

$t_1$  $t_2$  $t_3$  $t_4$  $t_5$  Zeit

*  **

Regelphasen

$t_1 + t_2 + t_3 + t_4 \triangleq T2$ (niederer Wert)
      in Beschreibung mit t2 bezeichnet

$t_5 \triangleq T1$ (hoher Wert)
(Substratfluß)

* Aktivieren einer hohen Substratflußrate mit neu berechneten Werten
** Aktivieren einer niederen Substratflußrate

# FIG.7

Batchphase 1        Regel Batch 1  → tB1

Batchphase 2        Regel Batch 2  → tB2

Batchphase 3        Regel Batch 3  → tB3

Batchphase 4        Regel Batch 4  → tB4

Regelungsphase 1     Regel Betrieb 1  → t21

Regelungsphase 2     Regel Betrieb 2  → t22

Regelungsphase 3     Regel Betrieb 3  → t23

Regelungsphase 4     Regel Betrieb 4  → t24

Regelungsphase 5     Regel Betrieb 5  → T1

→ T2 niederer Substratfluß

hoher Substratfluß

# FIG.8

Sollwert (Sollwertprofile)
für Phasenzeiten oder
Tastverhältnis

Phasenzeiten

Vom

Analysen -

und

Entscheidungsblock

Phase 4 -> 5

Phasenwechsel

Phase
5 -> 4

μ, x

Regelungsblock:
Berechnung des Pumpratenstartwertes — L1

Startwerte
der Pumprate

Pumpenratenprofil
hoher
Substratfluß — L2

Pumpenratenprofil
niedriger
Substratfluß — L3

# FIG.9

Experiment : HEFI71   vom : 09.06.1987   20:37

VD = Verdünnungsrate
PH = pH-Wert
BT = Biotrockenmassenkonzentration
TV = Testverhältnis

EP 0 345 588 A1

FIG .10

Experiment : HEFI 113    vom : 10.12.1987   10 : 14

OX = Gelöstsauerstoffkonzentration
BT = Biotrockenmassenkonzentration
u = spezifische Wachstumsrate
ZA = Zellmassenausbeute

EP 0 345 588 A1

# FIG .11

Experiment : HEFI113   vom : 10.12.1987   10:14

EP 0 345 588 A1

# FIG.12

Experiment: KLEB14   vom: 08.05.1988   20:10

EP 0 345 588 A1

FIG .13

Experiment: KLEB14   vom: 08.05.1988   20:10

CO = Kohlendioxidkonzentration
RP = Respirationsquotient
PK = Produktkonzentration

PK

RP

CO

Produktion [g/I]

RQ [-]

Vol% CO2 [%]

t [h]

EP 0 345 588 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | BIOTECHNOLOGY AND BIOENGINEERING, Band XXI, 1979, Seiten 323-328, John Wiley & Sons, Inc.; M.T. KLEIN et al.: "Periodic forcing of the phosphofructokinase oscillator in whole yeast cells" * Seiten 325-327 * | 1-11 | C 12 M 1/00 C 12 P 1/00 C 12 M 1/36 |
| X | FR-A-2 430 975 (I.C.I.) * Ansprüche; Figuren * | 1-11 | |
| A | US-A-3 647 633 (P.S. DAWSON) * Ansprüche; Figuren * | 1-11 | |
| A | EP-A-0 101 273 (THE BOARD OF TRUSTEES OF THE LELAND STANFORD JUNIOR UNIVERSITY) * Ansprüche * | 1 | |

RECHERCHIERTE SACHGEBIETE (Int. Cl.4)

C 12 M
C 12 P

Der vorliegende Recherchenbericht wurde für alle Patentansprüche erstellt

| Recherchenort | Abschlußdatum der Recherche | Prüfer |
|---|---|---|
| DEN HAAG | 21-09-1989 | COUCKE A.O.M. |